# EUROPEAN PATENT APPLICATION

(11) **EP 2 592 086 A1**
(43) Date of publication of application: **15.05.2013**
(21) Application number: 11766224.7
(22) Date of filing: 05.04.2011
(51) Int. Cl.: C07F 1/08, A61K 31/30, A61P 35/00

(54) **TELOMERASE INHIBITORS AND A METHOD FOR THE PREPARATION THEREOF**

(30) Priority: 09.04.2010 RU 2010113946
(71) Applicant: Gosudarstvennoe Uchebno-nauchnoe Uchrezhdenie Khimichesky Fakultet MGU Im. M.V. Lomonosova, Moscow 119991 (RU); Mazhuga, Aleksandr Georgievich, Moscow 107553 (RU); Zvereva, Marija Ehmil'evna, Moscow 127434 (RU)
(72) Inventor: AGRON, Leonid Aleksandrovich, Moscow 127550 (RU); BELOGLAZKINA, Elena Kimovna, Moscow 117587 (RU); VOROZHCOV, Nikolajj Igorevich, Moscow 119526 (RU); DONCOVA, Ol'ga Anatol'evna, Moscow 117630 (RU); ZYK, Nikolajj Vasil'evich, Moscow 119415 (RU); KISELEV, Fedor L'vovich, Moscow 117418 (RU); SKVORCOV, Dmitrijj Aleksandrovich, Moscow 127349 (RU); MAZHUGA, Aleksandr Georgievich, Moscow 107553 (RU); ZVEREVA, Marija Ehmil'evna, Moscow 127434 (RU)
(74) Representative: Winter, Brandl, Fürniss, Hübner, Röss, Kaiser, Polte - Partnerschaft
(86) International application number: PCT/RU2011/000223
(87) International publication number: WO 2011/126409

(57) **Abstract**

The invention relates to the field of organic and medicinal chemistry, and molecular biology, and concerns a method for the preparation of a new class of telomerase-inhibiting compounds, which can be utilized for studying telomerases and catalytic sub-units of telomerases, and reverse transcriptases, and foe studying and treating neoplastic and viral diseases. Telomerase-inhibiting coordination compounds of derivatives of imidazol-4-one are characterized by general formula (1).

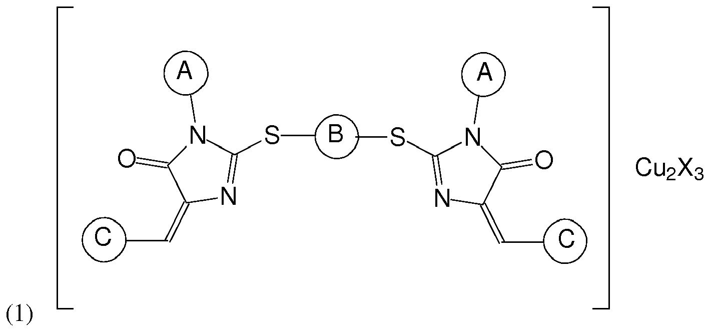

## Description

### Field of technology

The invention relates to the field of organic and medicinal chemistry, molecular biology and process for the preparation of a new class of compounds that inhibit telomerase, which can be used to test the catalytic subunit of telomerase and telomerase reverse transcriptase, for the study and treatment of cancer and viral diseases.

### Background

Telomerase - an enzyme that is required to compensate for the shortening of telomere length in eukaryotic cells. Telomeres consist of the typical tandem repeats (TTAGGG in humans), found at the ends of most eukaryotic chromosomes (Blackburn, "Structure and Function of Telomeres," Nature, 350:569-573, 1991). The number of repeats determines the length of telomeres. The proliferative potential of the cells linked to telomere length, because the stability and integrity of eukaryotic chromosomes depend on the dynamic of structural organization of telomeres (Baird DM. "Mechanisms of telomeric instability", Cytogenet Genome Res. 2008;122(3-4):308-14, 2009). When telomeres' shortening less than the critical length stability is violated, and then the cell dies. Telomeres play an important role in controlling the separation of chromosomes and are involved in the regulation of the cell cycle. With each cell division of a somatic cell, it loses about 60-100 bases from the ends of chromosomes. Telomeres are reduced; the cell eventually reaches a crisis and triggered apoptosis. In the body there are cells with unlimited potential for division. They (sex, stem cells, as well as malignant cells) have a process of compensation telomere shortening. The telomerase is responsible for this process. Telomerase is active in these cells and maintains telomere length above crisis levels. Telomerase - a specialized reverse transcriptase (for human hTERT), working in complex with its own ribonucleic acid (RNA). This is called telomerase RNA (human hTERC) and contains a site for the synthesis of telomeric DNA repeats (matrix region). In other words, to acquire the ability to divide indefinitely cell must activate the mechanism that supports telomere length above the critical level, ie telomerase. Thus, a significant level of telomerase activity was detected in more than 85% of tumors (Kim et al., "Specific Association of Human Telomerase Activity with Immortal Cells and Cancer," Science, 266:2011-2015, 1994). Telomerase activity is also present in stem bags of normal tissue, but at a lower level (Morin, "Is Telomerase a Universal Cancer Target?", J. Natl. Cancer Inst., 87:859-861, 1995). Thus, the presence of telomerase activity in tumor ensures that target, giving potentially good selectivity for tumor cells relative to normal tissue. Inhibition of telomerase has been proposed as a new approach to cancer therapy (the first of Morin, "Is Telomerase a Universal Cancer Target?", J. Natl. Cancer Inst., 87:859-861, 1995; Parkinson, "Do Telomerase Antagonists Represent a Novel Anti-Cancer Strategy?" Brit. J. Cancer, 73:1-4, 1996; Raymond et al., "Agents that target telomerase and telomeres," Curr Opinion Biotech.,7:583-591, 1996, a review of the modern state in Shay JW, Wright WE. Telomerase therapeutics for cancer: challenges and new directions. Nat Rev Drug Discov. 5(7):577-84, 2006).

The tertiary structure of the protein subunit of human telomerase catalytic remains unresolved at the moment, but the analysis of the primary structure shows that this protein is similar to other reverse transcriptase (Lingner et al., "Reverse Transcriptase Motifs in the Catalytic Subunit of Telomerase," Sci., 276:561-567, 1997), therefore its activity is suppressed by using reverse transcriptase inhibitors, such as: AZT (Strahl and Blackburn, "Effects of Reverse Transcriptase Inhibitors on Telomere Length and Telomerase Activity in Two Immortalized Human Cell Lines,"Mol. Cell. Biol.,16;53-65, 1996) and other nucleoside (Fletcher et al., "Human Telomerase Inhibition by 7-Deaza 2'-deoxypurine Nucleoside Triphosphates," Biochem, 35:15611-15617, 1996). Also, it was shown that inhibition of telomerase activity by the use of antisense sequence to the matrix portion of telomerase RNA, such as nucleic acids, fused to a peptide (Norton et al., "Inhibition of Human Telomerase Activity by Peptide Nucleic Acids," Nature Biotechnol., 14:615-619, 1996) and phosphorothioate oligonucleotides (Mata et al., "A Hexameric Phosphorothioate Oligonucleotide Telomerase Inhibitor Arrests Growth of Burkitt's Lymphoma Cells in Vitro and in Vivo, "Toxicol Appl. Pharmacol., 144:189-197, 1997). Antisense deoxyribonucleotide containing 185 nucleotides hTERC, was able to reduce the telomeres in HeLa cells after 23-26 cell divisions to a critical level and caused apoptosis. Another deoxyribonucleotide containing 2'-5'-adenylate (2-5A), so that not only bind, but also split hTERC, caused apoptosis in glioma, prostate cancer, cervical cancer, bladder and ovaries for 4-5 days. To increase the affinity for hTERC sequence and stability of oligonucleotide 2'-O-methyl RNA oligonucleotides were used. The most effective was the oligonucleotide GRN163 (Asai et al. A novel telomerase template antagonist (GRN163) as a potential anticancer agent. Cancer Research, 63:3931-3939, 2003). Cells cultured with this compound, were killed in 100 days. GRN163 inhibited telomerase activity at very low concentrations compared to the other oligonucleotides, GRN163L is the first inhibitor of telomerase, which entered into clinical practice. Preclinical studies have demonstrated the safety and efficacy of this inhibition. Safety and dose determination in patients refractory to other therapy, are under investigation. These studies have not been completed (USA, ClinicalTrials.gov, NCT00310895), but has shown the effectiveness of GRN163L - an unambiguous evidence that inhibition of telomerase - is the basis of anti-cancer therapy.

From WO99/01560 on 14.01.1999 (RU2000102361, the priority date of 1/7/1998) are also known other inhibitors of telomerase activity with oligonucleotide nature.

There are examples of coordination compounds of iron (III), zinc (II), nickel (II), manganese (III) and platinum (II) (Monchaud et al., "A hitchhiker's guide to G-quadruplex ligands", Org. Biomol. Chem., 6, 627, 2008). Most of coordination compounds contain porphyrin derivatives or condensed pyridine systems. Telomerase inhibition for them observed at values IC50-TRAP (IC50 - the concentration of inhibitor at which the enzyme activity is inhibited by 50%) from 0.12 to 30 µM.)

The closest analogue of the invention is an inhibitor of telomerase, which is a coordination compound of copper (II), containing a ligand-based porphyrin derivative (S. E. Evans, M. A. Mendez, K. B. Turner, L. R. Keating, R. T. Grimes, S. Melchoir and V. A. Szalai, J. Biol. Inorg. Chem., 2007, 12(8), 1235-1249). This compound selectively interact with quadruplex DNA, the value of IC50-TRAP in experiments on telomerase inhibition is 26 µM. The disadvantages of this telomerase inhibitor include the difficulty of synthesis of organic ligands and coordination compounds, as well as low IC50.

Mentioned drawbacks known inhibitors of telomerase can be overcome with the use of coordination compounds derived imidazole-4-one, described in more detail below with the accompanying illustrative material.

### Description of figures

Figure. 1. Structures of substituent's at position A in imidazol-4-one derivatives.
Figure. 2. Structures of substituent's at position B in imidazol-4-one derivatives.
Figure. 3. Structures of substituent's at position C in imidazol-4-one derivatives.
Figure 4. Description of the amplification of telomers (TRAP-analysis).
Figure. 5. Telomerase inhibition effect with different inhibitor concentration of (5Z, 5'Z)-2,2'-(etane-1,2-diyldisulfanyl)bis(5-(2-pyridilmethylen)-3-allyl-3,5-dihydro-4H-imidazol-4-one) complex with copper chloride.

### Disclosure of invention

According to the invention coordination compaunds on the basis of imidazole-4-ones can be used as telomerase inhibitors with general formula:

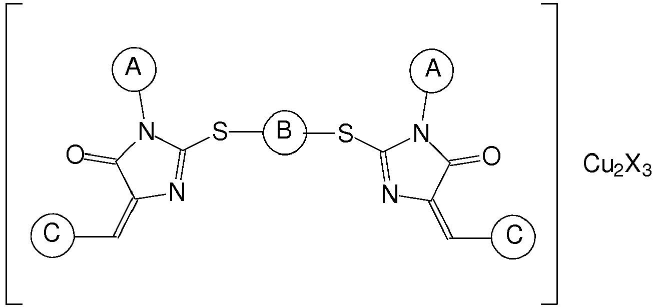

Where substituent A selected from group including aryl and alkyl substitutes, condensed aromatic groups, cyclopentyl, cyclohexyl, aliphatic substituent's, aliphatic substitutes with double bonds, aliphatic substitutes with triple bonds, CH₃NH- group, C₂H₅O(O)C-group, five membered heterocycles with one nitrogen atom, five membered heterocycles with two nitrogen atoms, six membered heterocycles, substitute B is absent or aliphatic substitute, substitute C is heteroaromatic substitute , attached to imidazol-4-one derivative via carbon atom and selected from the group including five membered saturated monocyclic heterocycles with 1, 2, 3 heteroatoms in cycle, selected from the group including N, O or S, 6-membered unsaturated monocyclic heteroaromatic substitutes with 1, 2, 3 heteroatoms in cycle, selected from the group including N, O or S, 8-, 9- or 10-membered unsaturated bicyclic heteroaromatic substitutes with 1, 2, 3 heteroatoms in cycle, selected from the group including N, O or S, X presents chloride Cl or nitrate NO₃.

In the preferred embodiment of the invention substituent A is unsubstituted or monosubstituted, or disubstituted with aryl group, in this case substitute R in aryl fragment choose from the group including halogens and alkyl groups.

In the preferred embodiment of the invention substituent A is unsubstituted or monosubstituted, or disubstituted with condensed aryl group, in this case substitute R in aryl fragment choose from the group including halogens and alkyl groups.

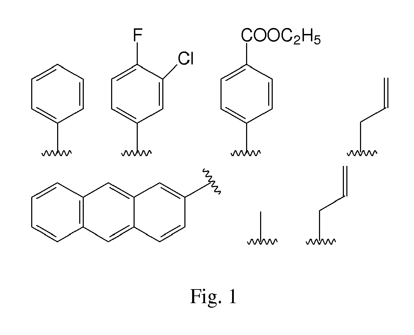

In the preferred embodiment of the invention substituent A was selected from the group including phenyl C₆H₅-, 3-chloro-4-fluorophenyl 3-Cl-4-F-C₆H₃-, 4-carbethoxyphenyl 4-C₂H₅O(O)CC₆H₄-, methyl CH₃-, allyl CH₂=CHCH₂-, 2-antranyl, propyl C₃H₇- (fig. 1).

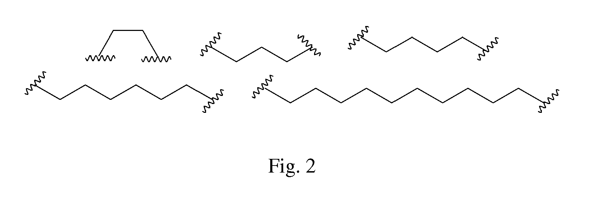

In the preferred embodiment of the invention substituent B was selected from the group including 1,2-ethandiyl -(CH₂)₂-, 1,3-propanediyl -(CH₂)₃-, 1,4-buthanediyl -(CH₂)₄-, 1,6-hexanediyl -(CH₂)₆-, 1,10-decanediyl -(CH₂)₁₀- (fig. 2).

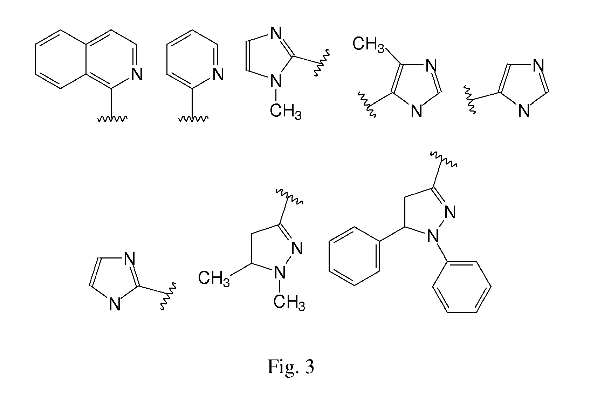

In the preferred embodiment of the invention substituent C was selected from the group including 2-quinolyl, 2-pyridyl, 1-methyl-2-imidazolyl, 4-methyl-5-imidazolyl, 5-imidazolyl, 2-imidazolyl, 1,5-dimethyl-3-pyrazolyl, 1,5-diphenyl-3-pyrazolyl (fig. 3).

In the method for the preparation of coordination compounds according to invention following steps were performed: solution of imidazol-4-one in DCM were slowly added to the solution of copper salt in methanol or acetonitrile, the precipitation of the coordination compound occurs.

In the case of coordination compounds cooper chloride or nitrate can be used.

Imidazol-4-ones with the general formula can be used:

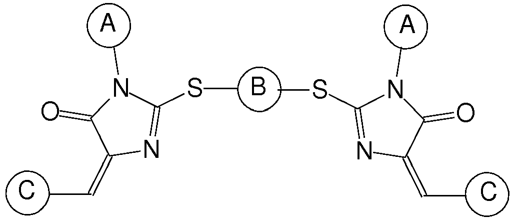

Where substituent A selected from group including aryl and alkyl substitutes, condensed aromatic groups, cyclopentyl, cyclohexyl, aliphatic substituent's, aliphatic substitutes with double bonds, aliphatic substitutes with triple bonds, CH₃NH- group, C₂H₅O(O)C-group, five membered heterocycles with one nitrogen atom, five membered heterocycles with two nitrogen atoms, six membered heterocycles, substitute B is absent or aliphatic substitute, substitute C is heteroaromatic substitute , attached to imidazol-4-one derivative via carbon atom and selected from the group including five membered saturated monocyclic heterocycles with 1, 2, 3 heteroatoms in cycle, selected from the group including N, O or S, 6-membered unsaturated monocyclic heteroaromatic substitutes with 1, 2, 3 heteroatoms in cycle, selected from the group including N, O or S, 8-, 9- or 10-membered unsaturated bicyclic heteroaromatic substitutes with 1, 2, 3 heteroatoms in cycle, selected from the group including N, O or S, X presents chloride Cl or nitrate NO₃.

In the preferred embodiment of the invention substituent A is unsubstituted or monosubstituted, or disubstituted with aryl group, in this case substitute R in aryl fragment choose from the group including halogens and alkyl groups.

In the preferred embodiment of the invention substituent A is unsubstituted or monosubstituted, or disubstituted with condensed aryl group, in this case substitute R in aryl fragment choose from the group including halogens and alkyl groups.

In the preferred embodiment of the invention substituent A was selected from the group including phenyl C₆H₅-, 3-chloro-4-fluorophenyl 3-Cl-4-F-C₆H₃-, 4-carbethoxyphenyl 4-C₂H₅O(O)CC₆H₄-, methyl CH₃-, allyl CH₂=CHCH₂-, 2-antranyl, propyl C₃H₇- (fig. 1).

In the preferred embodiment of the invention substituent B was selected from the group including 1,2-ethandiyl -(CH₂)₂-, 1,3-propanediyl -(CH₂)₃-, 1,4-buthanediyl -(CH₂)₄-, 1,6-hexanediyl -(CH₂)₆-, 1,10-decanediyl -(CH₂)₁₀- (fig. 2).

In the preferred embodiment of the invention substituent C was selected from the group including 2-quinolyl, 2-pyridyl, 1-methyl-2-imidazolyl, 4-methyl-5-imidazolyl, 5-imidazolyl, 2-imidazolyl, 1,5-dimethyl-3-pyrazolyl, 1,5-diphenyl-3-pyrazolyl (fig. 3).

Imidazole-4-ones can be synthesized by alkylation of the 3-substituted 2-thiohydantoins with α,ω-dibromoalkanes. For this purpose to dry 2-thiohydantoine derivative (2 equivalents) and dry K₂CO₃ (3 equivalents) in DMF at 0°C α,ω-dibromoalkane (1 equivalents) was added. Reaction mixture was stirred at 0°C for 2 h and 2 h at rt. After that 50 ml of water was added to the mixture. The resulting precipitate was filtered and washed with water and diethyl ether

The invention is illustrated by examples of alternative options of its performance.

### Example 1. Synthesis of (5Z, 5'Z)-2,2'-(ethan-1,2-diyldisulfanyldiyl)bis(5-(pyridine-2-ylmethylene)-3-methyl-3,5-dihydro-4H-imidazole-4-on)

(5Z,5'Z)-2,2'-(Ethan-1,2-diyldisulfanyldiyl)bis(5-(pyridine-2-ylmethylene)-3-methyl-3,5-dihydro-4H-imidazole-4-on) was obtained from 0,5 g (0,0025 mol) 2-thioxo-3-methyl-5-((Z)-pyridine-2-ylmethylene)-thetrahydro-4H-imidazole-4-on and 0,21 g (0,0013 mol) 1,2-dibromoetane. Yeld 0,40 g (76 %), mp=215°C.

¹H NMR (400MHz, CDCl₃, *δ*H ): 8,82 (d, 1H, H_{α}-Py, J=7,9Hz), 8,59 (d, 1H, H_{α}-Py, J=4,0Hz), 7,71 (td, 1H, H_{α}-Py, J₁=7,3Hz, J₂=2,3Hz), 7,23 (s, 1H, CH=), 7,13 (dd, 1H, H_{γ}-Py, J₁=7,5Hz, J₂=0,9Hz), 3,41 (t, 2H, S-CH₂, J=7,5Hz), 3,08 (s, 3H, N-CH₃).

IR (cm⁻¹): 1710 (C=O), 1670 (C=N), 1640 (C=C).

Element analysis: C₂₂H₂₀N₆O₂S₂ calculated C 56,88 %, H 4,34 %, N 18,09 %; found C 56,74 %, H 4,27 %, N 17,82 %.

### Example 2. Synthesis (5Z, 5'Z)-2,2'-(ethan-1,2-diyldisulfanyldiyl)bis(5-(pyridine-2-ylmethylene)-3-propyl-3,5-dihydro-4H-imidazole-4-on)

(5Z, 5'Z)-2,2'-(ethan-1,2-diyldisulfanyldiyl)bis(5-(pyridine-2-ylmethylene)-3-propyl-3,5-dihydro-4H-imidazole-4-on) was obtained from 0,5 g (0,002 mol) 2-thioxo-3-propyl-5-((Z)-pyridine-2-ylmethylene)-thetrahydro-4H-imidazole-4-on and 0,19 g (0,001 mol) 1,2-dibrometana. Yeld 0,43 g (82%), mp=152°C.

¹H NMR (400MHz, CDCl₃, *δ*H ): 8,69(d, J=8,0Hz, 1H, H_{α}-Py,), 8,65 (d, J=4,7, 1H, H_{β'}-Py,), 7,63 (td, J₁=7,4Hz, J₂=2,0Hz, 1H, H_{β}-Py,), 7,19 (dd, J₁=7,5Hz, J₂=0,9Hz, 1H, H_{γ}-Py,), 7,12 (s, 1H, CH=), 7,12 (s, 1H, CH=), 3,93 (s, 2H, S-CH₂), 3,60 (t, J=7,5 Hz, 2H, CH₂N), 1,72 (m, 2H, CH₂), 0,96 (t, J=7,5, 3H, CH₃).

IR (cm⁻¹)1705(C=O), 1675(C=N), 1640(C=C).

Element analysis: C₂₆H₂₈N₆O₂S₂ calculated C 59,98% H 5,42% N 16,14%; found C 59,34% H 5,17% N15,88%.

### Example 3. Synthesis (5Z, 5'Z)-2,2'-(ethan-1,2-diyldisulfanyldiyl)bis(5-(pyridine-2-ylmethylene)-3-allyl-3,5-dihydro-4H-imidazole-4-on)

(5Z,5'Z)-2,2'-(ethan-1,2-diyldisulfanyldiyl)bis(5-(pyridine-2-ylmethylene)-3-allyl-3,5-dihydro-4H-imidazole-4-on) was obtained from 0,5 g (0,002 mol) 2-thioxo-3-allyl-5-((Z)-pyridine-2-ylmethylene)-thetrahydro-4H-imidazole-4-on and 0,19 g (0,001 mol) 1,2-dibrometan. Yeld 0,46 g (92%), mp=187°C.

¹H NMR (400MHz, CDCl₃, δH ): 8,65(m, 2H, H_{α}-Py + H_{β'}-Py,), 7,62 (t, J=7,5Hz, 1H, H_{β}-Py,), 7,19 (m, 1H, H_{γ}-Py,), 7,14 (s, 1H, CH=), 7,12 (s, 1H, CH=), 5,82 (m, 1H, CH=), 5,23 (m., 2H, CH₂=), 4,23 (m, 2H, CH₂N) 3,89 (s, 2H, S-CH₂).

IR(cm-1): 1720(C=O), 1680(C=N), 1640(C=C).

Element analysis: C₂₆H₂₆N₆O₂S₂ calculated C 60,44% H 4,68% N 16,27%; found C 60,14% H 4,48% N 16,03%.

### Example 4. Synthesis (5Z, 5'Z)-2,2'-(ethan-1,2-diyldisulfanyldiyl)bis(5-(pyridine-2-ylmethylene)-3-phenyl-3,5-dihydro-4H-imidazole-4-on)

(5Z,5'Z)-2,2'-(ethan-1,2-diyldisulfanyldiyl)bis(5-(pyridine-2-ylmethylene)-3-phenyl-3,5-dihydro-4H-imidazole-4-on) was obtained from 0,3 g (0,001 mol) 2-thioxo-3-phenyl-5-((Z)-pyridine-2-ylmethylene)-thetrahydro-4H-imidazole-4-on and 0,1 g (0,001 mol) 1,2-dibrometan. Yeld 0,46 g (92%), mp=259°C

¹H NMR (400MHz, CDCl₃, δH ): 8,75(d, 2H, H_{α}-Py, J=7,9Hz), 8,66(d, 2H, H_{β'}-Py, J=4,0Hz), 7,81(td, 2H, H_{β}-Py, J₁=7,3Hz, J₂=2,3Hz), 7,42(m, 6H, H-Ph), 7,29(m, 4H, H-Ph), 7,11(td, 2H, H_{γ}-Py, J₁=7,5Hz, J₂=1,0Hz), 7,18(s, 2H, CH=), 3,11(t, 4H, S-CH₂-, J=7,5Hz).

IR (cm⁻¹): 1710(C=O), 1670(C=N), 1640(C=C).

Element analysis: C₃₂H₂₄N₆S₂O₂ calculated C% 65,31, H% 4,08, N% 14,29; found C% 65,28, H% 4,10, N% 14,11.

### Example 5. Synthesis (5Z, 5'Z)-2,2'-(ethan-1,2-diyldisulfanyldiyl)bis(5-(pyridine-2-ylmethylene)-3-nuphthyl-3,5-dihydro-4H-imidazole-4-on)

(5Z,5'Z)-2,2'-(ethan-1,2-diyldisulfanyldiyl)bis(5-(pyridine-2-ylmethylene)-3-nuphthyl-3,5-dihydro-4H-imidazole-4-on) was obtained from 0,3 g (0,001 mol) 2-thioxo-3-nuphthyl-5-((Z)-pyridine-2-ylmethylene)-thetrahydro-4H-imidazole-4-on and 0,1 g (0,001 mol) 1,2-dibrometan. Yeld 0,19 g (68%), mp=267°C

¹H NMR (400MHz, CDCl₃, δH): 9,12 (d, J=8,8 Hz, 1H, HetAr), 8,30 (d, J=8,7 Hz, 1H, HetAr), 8,20 (d, J=8,3 Hz, 1H, HetAr), 8,15 (d, J=8,3 Hz, 1H, Ar), 7,95 (m, 1H, Ar), 7,78 (d, J=8,1 Hz, 1H, HetAr), 7,69 (t, J=7,1 Hz, 1H, HetAr), 7,61 (m, 1H, Ar), 7,53(m, 6H, HetAr + CH= + Ar), 6,91 (s, 2H, CH=), 3,76 (s, 4H, CH₂-S).

IR (cm⁻¹): 1710(C=O), 1670(C=N), 1640(C=C).

### Example 6. Synthesis (5Z, 5'Z)-2,2'-(ethan-1,2-diyldisulfanyldiyl)bis(5-(pyridine-2-ylmethylene)-3-antrnyl-3,5-dihydro-4H-imidazole-4-on)

((5Z,5'Z)-2,2'-(ethan-1,2-diyldisulfanyldiyl)bis(5-(pyridine-2-ylmethylene)-3-antranyl-3,5-dihydro-4H-imidazole-4-on) was obtained from 0,4 g (0,001 mol) 2-thioxo-3-antranyl-5-((Z)-pyridine-2-ylmethylene)-thetrahydro-4H-imidazole-4-on and 0,1 g (0,001 mol) 1,2-dibrometan. Yeld 0,21 g (81%), mp=249°C

¹H NMR (400MHz, CDCl₃, δH ): 8,73 (d, J=7,9 Hz, 2H, Ar), 8,69 (d, J=5,1 Hz, 1H, H_{α'}-Py), 8,45 (m, 4H, Ar), 8,13 (d, J=8,8 Hz, 2H, H_{β}-Py), 8,01 (m, 3H, Ar), 7,76 (t, J=7,6 Hz, 1H, H_{β'}-Py), 7,51 (m, 4H, Ar), 7,47 (dd, J₁=6,8 Hz, J₂=1,4 Hz, 2H, H_{γ}-Py)), 7,25 (m, 2H, Ar), 6,94 (s, 2H, CH=), 3,83 (s, 4H, CH₂-S).

IR(cm⁻¹): 1730(C=O), 1680(C=N), 1620(C=C).

Element analysis: C₄₈H₃₂N₆O₂S₂ calculated C 73,08% H 4,09% N 10,65%; found C 73,35% H 4,82% N 10,13%.

### Example 7. Synthesis (5Z, 5'Z)-2,2'-(butan-1,2-diyldisulfanyldiyl)bis(5-(pyridine-2-ylmethylene)-3-allyl-3,5-dihydro-4H-imidazole-4-on)

(5Z,5'Z)-2,2'-(butan-1,2-diyldisulfanyldiyl)bis(5-(pyridine-2-ylmethylene)-3-allyl-3,5-dihydro-4H-imidazole-4-on) was obtained from 0,05 g (0,002 mol) 2-thioxo-3-allyl-5-((Z)-pyridine-2-ylmethylene)-thetrahydro-4H-imidazole-4-on and 0,22 g (0,001 mol) 1,4-dibrombutan. Yeld 0,40 g (76%), mp=198°C

¹H NMR (400MHz, CDCl₃, δH ): 8,72(d, J=8,1Hz, 2H, H_{α'}-Py), 8,65(d, J=3,8Hz, 2H, H_{β}-Py), 7,63(td, J₁=8,3Hz, J₂=4,6Hz, 2H, H_{γ}-Py), 7,05(dd, J₁=4,7 Hz, J₂=1,2 Hz, 2H, H_{β'}-Py,), 7,12(s, 2H, CH=), 5,80 (m, 2H, =CH-) 5,25 (m, 4H, CH₂=), 4,22 (d, J=7,1 Hz, 4H, -CH₂-N), 3,45(d, J=6,7 Hz, 4H, -CH₂-S), 2,07 (m, 4H, -CH₂-).

IR (cm⁻¹): 1710(C=O), 1670(C=N), 1640(C=C).

Element analysis: C₂₈H₂₈N₆O₂S₂ calculated C 61,74% H 5,18% N 15,43%; found C 61,74% H 5,18% N 15,43%.

### Example 8. Synthesis (5Z, 5'Z)-2,2'-(butan-1,2-diyldisulfanyldiyl)bis(5-(pyridine-2-ylmethylene)-3-phenyl-3,5-dihydro-4H-imidazole-4-on)

(5Z,5'Z)-2,2'-(butan-1,2-diyldisulfanyldiyl)bis(5-(pyridine-2-ylmethylene)-3-phenyl-3,5-dihydro-4H-imidazole-4-on) was obtained from 0,3 g (0,001 mol) 2-thioxo-3-phenyl-5-((Z)-pyridine-2-ylmethylene)-thetrahydro-4H-imidazole-4-on and 0,12 g (0,0005 mol) 1,4-dibrombutan. Yeld 0,18 g (78%), mp=249°C

¹H NMR (400MHz, CDCl₃, δH ): 8,75(d, J=7,9 Hz, 2H, H_{α}-Py), 8,66(d, J=4,0 Hz, 1H, H_{β'}-Py), 7,81(td, J₁=7,3 Hz, J₂=2,3 Hz, 2H, H_{β}-Py), 7,42(m, 6H, H-Ph), 7,29(m, 4H, H-Ph), 7,11(td, J₁=7,5 Hz, J₂=1,0 Hz, 2H, H_{γ}-Py), 7,18(s, 2H, CH=), 3,11(t, J=7,5 Hz, 4H, S-CH₂-,) 1,86(qw, J=7,6Hz, 4H, -CH₂-).

IR (cm⁻¹): 1720(C=O), 1670(C=N), 1640(C=C).

Element analysis: C₃₄H₂₈N₆O₂S₂ calculated C 66,21% H 4,58% N 13,63%; found C 66,12% H 4,76% N 13,20%.

### Example 9. Synthesis (5Z, 5'Z)-2,2'-(hexan-1,2-diyldisulfanyldiyl)bis(5-(pyridine-2-ylmethylene)-3-allyl-3,5-dihydro-4H-imidazole-4-on)

(5Z,5'Z)-2,2'-(hexan-1,2-diyldisulfanyldiyl)bis(5-(pyridine-2-ylmethylene)-3-allyl-3,5-dihydro-4H-imidazole-4-on) was obtained from 0,5 g (0,002 mol) 2-thioxo-3-allyl-5-((Z)-pyridine-2-ylmethylene)-thetrahydro-4H-imidazole-4-on and 0,22 g (0,001 mol) 1,4-dibromhexan. Yeld 0,40 g (81%), mp=171°C

¹H NMR (400MHz, CDCl₃, δH ): 8,75 (d, J=8,2Hz, 2H, H_{α}-Py), 8,67(d, J=4,1Hz, 2H, H_{β}-Py), 7,68(t, J=7,8 Hz, 21H, H_{γ}-Py), 7,16(m, 2H, H_{β'}-Py), 7,13(s, 2H, CH=), 5,83(m, 2H, - CH=), 5,25(m, 4H, CH₂=), 4,15(d, J=5,9, 4H, -CH₂-N), 3,38 (t, J=7,0 Hz, 4H, CH₂-S), 1,91 (m, 4H, -CH₂-), 1,60 (m, 4H, -CH₂-).

IR (cm⁻¹): 1710(C=O), 1670(C=N), 1640(C=C).

Element analysis: C₃₀H₃₂N₆O₂S₂ calculated C 62,91% H 5,63% N 14,67%; found C 62,91% H 5,63% N 14,67%.

### Example 10. Synthesis (5Z, 5'Z)-2,2'-(hexan-1,2-diyldisulfanyldiyl)bis(5-(pyridine-2-ylmethylene)-3-phenyl-3,5-dihydro-4H-imidazole-4-on)

(5Z,5'Z)-2,2'-(hexan-1,2-diyldisulfanyldiyl)bis(5-(pyridine-2-ylmethylene)-3-phenyl-3,5-dihydro-4H-imidazole-4-on) was obtained from 0,3 g (0,001 mol) 2-thioxo-3-phenyl-5-((Z)-pyridine-2-ylmethylene)-thetrahydro-4H-imidazole-4-on and 0,13 g (0,0005 mol) 1,4-dibromhexan. Yeld 0,19 g (64%), mp=240°C

¹H NMR (400MHz, CDCl₃, δH ): 8,77(d, J=7,9Hz, 2H, H_{α}-Py,), 8,66(d, J=3,9Hz ,1H, H_{β'}-Py), 7,75(td, J₁=7,3Hz, J₂=2,3Hz, 2H, H_{β}-Py), 7,45(m, 6H, H-Ph), 7,30(m, 4H, H-Ph), 7,17(s, 2H, CH=), 7,13(dd, J₁=7,5Hz, J₂=0,9Hz, 2H, H_{γ}-Py), 3,32(t-, J=7,5Hz, 4H, S-CH₂), 1,86(m, 4H, -CH₂-), 1,54(m, 4H, -CH₂-).

IR (cm⁻¹): 1700(C=O), 1670(C=N), 1630(C=C).

Element analysis: C₃₆H₃₂N₆S₂O₂ calculated C% 67,06, H% 5,00, N% 13,03; found C% 66,71, H% 5,04, N% 12,65.

### Example 11. Synthesis (5Z, 5'Z)-2,2'-(ethan-1,2-diyldisulfanyldiyl)bis(5-(quinole-2-ylmethylene)-3-phenyl-3,5-dihydro-4H-imidazole-4-on)

(5Z,5'Z)-2,2'-(ethan-1,2-diyldisulfanyldiyl)bis(5-(quinole-2-ylmethylene)-3-phenyl-3,5-dihydro-4H-imidazole-4-on) was obtained from 0,5 g (0,0015 mol) 2-thioxo-3-phenyl-5-((Z)-quinole-2-ylmethylene)-thetrahydro-4H-imidazole-4-on and 0,15 g (0,0008 mol) 1,4-dibromethan. Yield 0,75 g (81%), mp=140°C

¹H NMR (400MHz, CDCl₃, δH): 8,82 (d, J=9,1 Hz, 2H, HetAr), 8,05 (m, 4H, HetAr), 7,72 (m, 4H, HetAr), 7,53 (m, 2H, HetAr), 7,29 (s, 2H, CH=), 4,00(s, 4H, -CH₂-S), 3,64(t, J=7,4 Hz, 4H, CH₂-N), 1,75 (m, 4H, CH₂), 1,01 (t, J=7,3, CH₃-).

IR (cm⁻¹): 1715(C=O), 1670(C=N), 1640(C=C).

Element analysis: C₃₈H₃₈N₂O₂S₂ calculated C 73,75% H 6,19% N 4,53%; found C 73,13% H 6,53% N 4,88%.

### Example 12. Synthesis (5Z, 5'Z)-2,2'-(ethan-1,2-diyldisulfanyldiyl)bis(5-(quinole-2-ylmethylene)-3-allyl-3,5-dihydro-4H-imidazole-4-on)

(5Z,5'Z)-2,2'-(ethan-1,2-diyldisulfanyldiyl)bis(5-(quinole-2-ylmethylene)-3-allyl-3,5-dihydro-4H-imidazole-4-on) was obtained from 0,5 g (0,0015 mol) 2-thioxo-3-allyl-5-((Z)-quinole-2-ylmethylene)-thetrahydro-4H-imidazole-4-on and 0,15 g (0,0008 mol) 1,4-dibromethan. Yeld 0,70 g (78%), mp=180°C

¹H NMR (400MHz, CDCl₃, δH ): 8,82 (d, J=9,0 Hz, 2H, HetAr), 8,05 (m, 4H, HetAr), 7,73 (m, 4H, HetAr), 7,57 (m, 2H, HetAr), 7,32 (s, 2H, CH=), 5,88 (m, 2H, CH=), 5,30 (m, 4H, CH₂=), 4,30 (d, J=5,6Hz, 4H, -CH₂-N), 3,97(s, 4H, CH₂-S).

IR (cm⁻¹): 1710(C=O), 1670(C=N), 1640(C=C).

Element analysis: C₃₈H₃₄N₂O₂S₂ calculated C 74,24% H 5,57% N 4,56%; found C 74,42% H 5,14% N 4,89%.

### Example 13. Synthesis (5Z, 5'Z)-2,2'-(ethan-1,2-diyldisulfanyldiyl)bis(5-(quinole-2-ylmethylene)-3-phenyl-3,5-dihydro-4H-imidazole-4-on)

(5Z,5'Z)-2,2'-(ethan-1,2-diyldisulfanyldiyl)bis(5-(quinole-2-ylmethylene)-3-phenyl-3,5-dihydro-4H-imidazole-4-on) was obtained from 0,5 g (0,0015 mol) 2-thioxo-3-phenyl-5-((Z)-quinole-2-ylmethylene)-thetrahydro-4H-imidazole-4-on and 0,14 g (0,0007 mol) 1,4-dibromethan. Yeld 0,41 g (78%), mp=239°C.

¹H NMR (400MHz, CDCl₃, δH): 8,84 (d, J=8,6 Hz, 2H, HetAr), 8,05 (d, J=8,8 Hz, 2H, HetAr), 7,97 (d, J=8,3 Hz, 2H, HetAr), 7,73 (m, 4H, HetAr), 7,45 (m, 14H, Ar + CH= + HetAr), 3,93 (s, 4H, -CH₂-S).

IR (cm⁻¹): 1710(C=O), 1670(C=N), 1640(C=C).

Element analysis: C₄₄H₃₄N₂O₂S₂ calculated C 76,94% H 4,99% N 4,08%; found C 76,67% H 4,13% N 3,98%.

### Example 14. Synthesis of coordination compounds from imidazole-4-on derivatives

To solution of 0,0001 mol of alkyl derivatives of 2-thiohidantoin (derivatives the imidazole-4-on) in 2-3 ml of a methylene chloride 2 ml of methanol is added by for achievement of stratification. Then slowly, on drops, within not less than 2 minutes flow solution of 0,0002 mol of salt of copper in 2-3 ml of methanol. A reactionary mix densely close and leave before formation of a precipitat.

In case of (5Z, 5'Z)-2,2'-(ethan-1,2-diyldisulfanyldiyl)bis(5-(2-pyridine-2-ylmethylene)-3-allyl-3,5-dihydro-4H-imidazole-4-on) complexes with copper nitrate acetonitrile was used as a solvent. Reaction mixture was let until formation of a precipitat.

### Complex (5Z,5'Z)-2,2'-(ethan-1,2-diyldisulfanyldiyl)bis(5-(pyridine-2-ylmethylene -3-methy-3,5-dihydro-4H-imidazole-4-on) with CuCl₂•2H₂O

From 0,05g (5Z,5'Z)-2,2'-(ethan-1,2-diyldisulfanyldiyl)bis(5-(pyridine-2-ylmethylene - 3-methy-3,5-dihydro-4H-imidazole-4-on) and 0.03g CuCl₂·6H₂O receive 0,04 g (54 %) complex of brown color.

Element analysis: C₂₂H₂₀N₆O₂S₂*CuCl₂*CuCl calculated C 37,86% H 2,89% N 12,04%; found C 37,04% H 2,57% N 12,22%.

### Complex (5Z,5'Z)-2,2'-(ethan-1,2-diyldisulfanyldiyl)bis(5-(pyridine-2-ylmethylene -3-propyl-3,5-dihydro-4H-imidazole-4-on) with CuCl₂•2H₂O

From 0,05g (5Z,5'Z)-2,2'-(ethan-1,2-diyldisulfanyldiyl)bis(5-(pyridine-2-ylmethylene - 3-propyl-3,5-dihydro-4H-imidazole-4-on) and 0.03g CuCl₂·6H₂O receive 0,03 g (45%) complex of brown color.

Element analysis: C₂₆H₂₈N₆O₂S₂*CuCl₂*CuCl calculated C 41,41% H 3,74% N 11,14%; found C 41,63% H 3,87% N 11,86%.

### Complex (5Z,5'Z)-2,2'-(ethan-1,2-diyldisulfanyldiyl)bis(5-(pyridine-2-ylmethylene -3-allyl-3,5-dihydro-4H-imidazole-4-on) with CuCl₂•2H₂O

From 0,05g (5Z,5'Z)-2,2'-(ethan-1,2-diyldisulfanyldiyl)bis(5-(pyridine-2-ylmethylene - 3-allyl-3,5-dihydro-4H-imidazole-4-on) and 0.03g CuCl₂·6H₂O receive 0,03 g (40%) complex of brown color.

Element analysis: C₂₆H₂₄N₆O₂S₂* CuCl₂*CuCl calculated C 41,63% H 3,23% N 11,15%; found C 41,36% H 3,23% N 11,85%.

### Complex (5Z,5'Z)-2,2'-(ethan-1,2-diyldisulfanyldiyl)bis(5-(pyridine-2-ylmethylene -3-phenyl-3,5-dihydro-4H-imidazole-4-on) with CuCl₂•2H₂O

From 0,05g (5Z,5'Z)-2,2'-(ethan-1,2-diyldisulfanyldiyl)bis(5-(pyridine-2-ylmethylene - 3-phenyl-3,5-dihydro-4H-imidazole-4-on) and 0.03g CuCl₂·6H₂O receive 0,03 g (55%) complex of brown color.

Element analysis: C₃₂H₂₄N₆O₂S₂*CuCl₂*CuCl calculated C 46,75% H 2,94% N 10,22%; found C 46,67% H 2,14% N 10,77%.

### Complex (5Z,5'Z)-2,2'-(butan-1,2-diyldisulfanyldiyl)bis(5-(pyridine-2-ylmethylene -3-allyl-3,5-dihydro-4H-imidazole-4-on) with CuCl₂•2H₂O

From 0,05g (5Z,5'Z)-2,2'-(butan-1,2-diyldisulfanyldiyl)bis(5-(pyridine-2-ylmethylene - 3-allyl-3,5-dihydro-4H-imidazole-4-on) and 0.03g CuCl₂·6H₂O receive 0,04 g (59%) complex of brown color.

Element analysis: C₂₈H₂₈N₆O₂S₂*CuCl₂*CuCl is calculated C 43,22% H 3,63% N 10,80%; is found C 43,45% H 3,33% N 10,30%.

### Complex (5Z,5'Z)-2,2'-(butan-1,2-diyldisulfanyldiyl)bis(5-(pyridine-2-ylmethylene -3-phenyl-3,5-dihydro-4H-imidazole-4-on) with CuCl₂•2H₂O

From 0,05g (5Z,5'Z)-2,2'-(butan-1,2-diyldisulfanyldiyl)bis(5-(pyridine-2-ylmethylene - 3-phenyl-3,5-dihydro-4H-imidazole-4-on) and 0.03g CuCl₂·6H₂O receive 0,03 g (49%) complex of brown color.

Element analysis: C₃₄H₂₈N₆O₂S₂*CuCl₂*CuCl calculated C 48,03% H 3,32% N 9,88% found C 48,49% H 2,60% N 9,10%.

To test the inhibition of telomerase compounds prepared in this way, we used the method of amplification added telomeric repeats (TRAP-analysis). TRAP-analysis is a standard method for determining the activity of telomerase, due to some modification this method could be semi quantitative. The choice of method for detection of telomerase activity was determined by its wide coverage in the world's literature, almost sensitivity, as well as the availability of equipment and reagents.

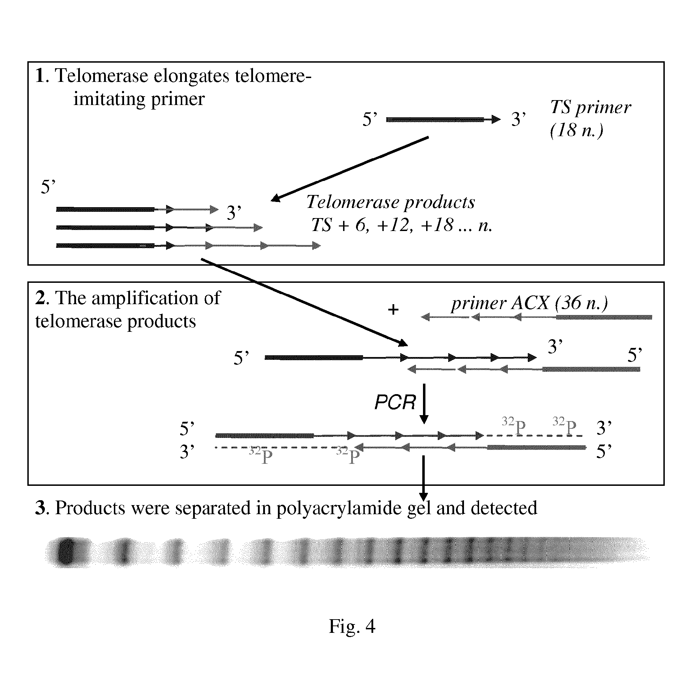

Telomeric repeats amplification protocol can be divided into three main steps: primer extension, amplification of the resulting product (s) and detection. In step lengthening telomeric repeats added to an oligonucleotide by telomerase in the cell extract. Telomerase recognizes it as a substrate (TS). In step of amplification of extension products of TS oligonucleotide by telomerase false signals may appear due to amplification of telomeres of chromosomes possibly have contained in the cell extract. To avoid this, the 5'-end of the oligonucleotide TS has no telomeric sequence, which prevents it to contact with telomeres, but is recognized as a telomerase substrate. Since human telomerase adds a series repeats with six nucleotides, the resulting lengthening of TS oligonucleotide by telomerase set of DNA fragments that differ in length synthesized. This is followed by a step increase in the number of products with specific primers by PCR with nucleotides containing radioactive or fluorescent label for detection Next is detection (Fig. 4), usually by electrophoretic separation and subsequent scans. Primers TS and ACX (Table 1) were used in the TRAP-analysis, ACX is at the 5'-end of the telomeric no appendage of 6 nucleotides, due to this also does not form a dimer with a telomerase substrate. By using these primers integrated labels is in proportion to the namber of repeats added by telomerase.

**Table 1. Sequences of oligonucleotides used in the measurement of telomerase activity.**

| Name | Sequence of oligonucleotides |
|---|---|
| TS | 5'-AATCCGTCGAGCAGAGTT-3' |
| ACX | 5'-GCGCGG(CTTACC)3CTAACC-3' |

Amount of PCR product is weakly dependent on the number of original matrix in the reaction mix due to saturation in PCR, so we can not estimate the amount of telomerase product by the intensity of its signal in the picture. With the introduction of a set of telomerase products in PCR they all are amplified. So we can use the number of added by telomerase repeats as a criterion of its activity.

The first step was the cultivation of human cancer cell lines to isolate the active extracts required for testing telomerase activity. To do this, cells of cervical carcinoma lines: SiHa, C33A, CaSki and HeLa were grown in standard medium DMEM, containing 10% fetal calf serum (FCS), 4mM L-glutamine, 1mM sodium pyruvate, streptomycin / penicillin at a concentration of 100 µg / ml and 100 U / ml, respectively, at 37 ° C under 5% CO2. For reseeding cell monolayer cells were washed PBS (10 mM Na2HPO4, 2 mM KH2PO4, 137 mM NaCl, 2 mM KCl), added standard solution trypsin: EDTA (Sigma) and placed in a CO2 incubator for 3-5 minutes, add a medium with FCS and suspended by pipetting, cells dissipates into the necessary number of culture flasks. After the formation of the monolayer cell lines were washed off from the substrate with a solution of trypsin and pelleted by centrifugation (10 min., 2000g), washed twice with buffer PBS. Cells were resuspended in lysis buffer (10 mM Tris-HCl and 10 mM HEPES-KOH, pH 7.5, 1.0 mM MgCl2, 1 mM EGTA, 5 mM β-mercaptoethanol, 5% glycerol, 0,5% CHAPS, 0, 1 mM PMSF), 1 ml for 0.3-10 million cells, depending on the desired concentration. They were incubated for 30 minutes on ice and centrifuged for 10 min at 4 ° C at 15,000 rev / min, and the supernatant solution was collected. The extract was divided into aliquots of 10 µl and frozen in liquid nitrogen. After it the analysis of telomerase activity by TRAP-test was done. In the first step a mixture of N1 was prepared: 49 µl mixture of TRAP-containing 1x buffer (1X TRAP-buffer: 20 mM HEPES-KOH pH 8,3, 1,5 mM MgCl2, 63 mM KCl, 1mM EGTA, 0,1 mg / ml BSA, 0,005% v / v Tween-20) with 20 µM dNTP , 1,6 µM oligonucleotide TS, 1 µl of the solution of substance in DMSO and cell extracts of cell lines or tissues. The reaction mixture was incubated for 30 min at 30 ° C. In the second step, in the mixture next ingredients were added: 2 u of Taq-DNA polymerase ("Helicon"), 0.1 mg oligonucleotide ACX. PCR was performed as follows: 35 s 94 ° C, 35 s to 50° C , 90 s to 72 ° C (30 cycles of thermal cycler Mastercycler ("Eppendorf")). 15 µl of solution of products and 2.5 µl loading buffer 6x DNA loading dye ("Fermentas", 10 mM Tris-HCl, pH 7,6, 0,03% bromophenol blue, 0.03% ksilenotsianola, 60% glycerol, 60 mM EDTA ) was applied to 20% polyacrylamide gel (acrylamide: BIS-acrylamide 1:19 10% TVE1h, TEMED 0,1%, 0,1% ammonium persulfate). As the electrode buffer TBE 1x (0,1 M Tris, 0,1 M H3BO3, 2 mM Na2EDTA) was used. Electrophoresis was performed until Xylene dye will not pass 10-20 cm. The gel was stained with a solution of SYBR Green (10,000 x concentrate in DMSO company Sigma-Aldrich, diluted 10,000 times 0.1 M buffer Tris-HCl, pH 8,5). Result was detected by fluorescence scanning of the gel. As a control that substances inhibit the RNA-dependent DNA polymerase (reverse transcriptase), namely telomerase, and not inhibit the DNA polymerase, which is used in the analysis in the second step of TRAP-assay to amplify the signal by PCR 1 µl solution of the drug was not poured into mixture on the first step but was added with oligonucleotide ACX for PCR on the second step. This control reaction was carried out simultaneously for each test.

To determine the IC50 (concentration of the substance on which the inhibition of telomerase activity is 50%) the reaction was carried out for different concentrations of drugs. IC50 value is given in Table 2.

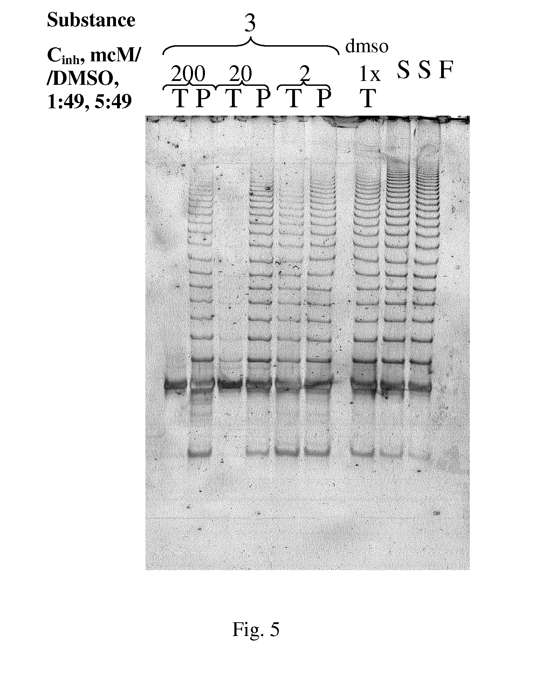

To better define the IC50 conducted separate repeated measurement of inhibiting substances with additional dilutions. An example of such an analysis for the drug with the highest inhibitory activity is shown in FIG. 5. Experimental images were analyzed using Image Qvant and the intensity of the bands corresponding to the same lengthening of TS by telomerase in the tracks T and P for each concentration of the drug was compared.

**Table 2. Testing of inhibition of telomerase activity by TRAP-assay for series of substances.**

| Number | Substances | Inhibition of telomerase , IC₅₀ |
|---|---|---|
| 1 | 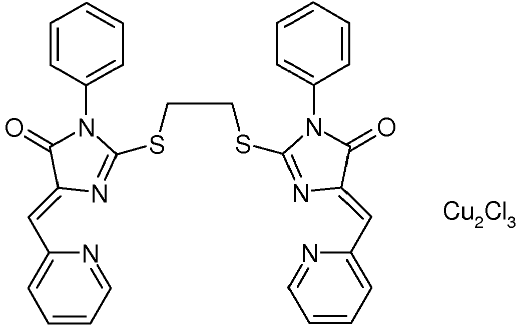 | 7 µM^{*} |
| 2 | 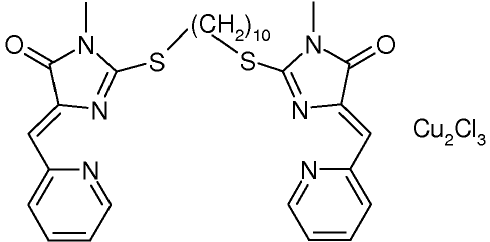 | 14 µM^{*} |
| 3 | 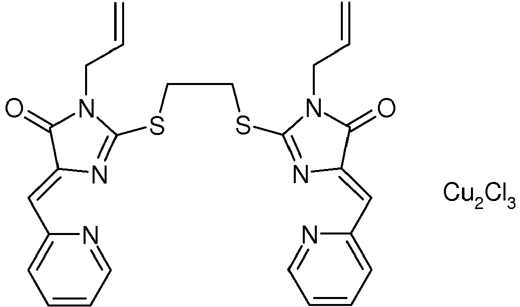 | 2 µM |
| 4 | 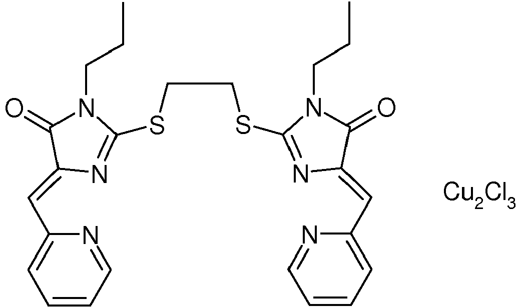 | 4 µM |
| 5 | 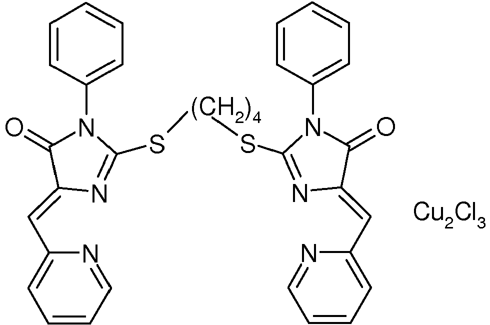 | 4 µM |
| 6 | 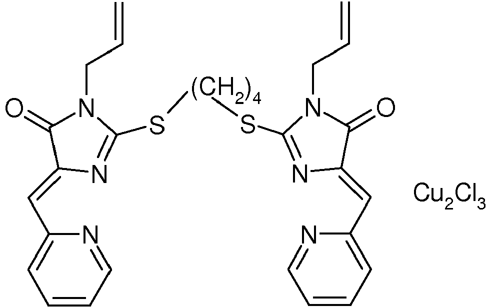 | 20 µM |
| 7 | 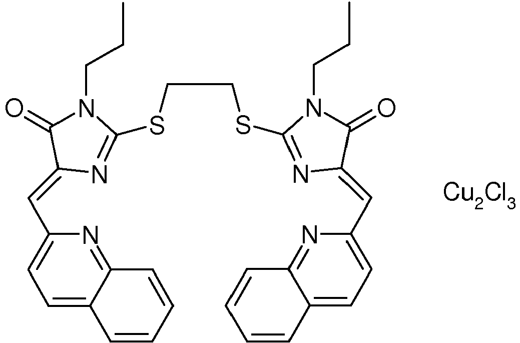 | 20 µM |

| | | |
|---|---|---|
| * For these substances, the value corresponds to the inhibitory effect for 15% of a saturated solution (IC50 is not measurable due to the low solubility of the initial preparations). | | |

## Claims

1. Coordination compounds on the basis of imidazol-4-ones as telomerase inhibitors, with general formula: 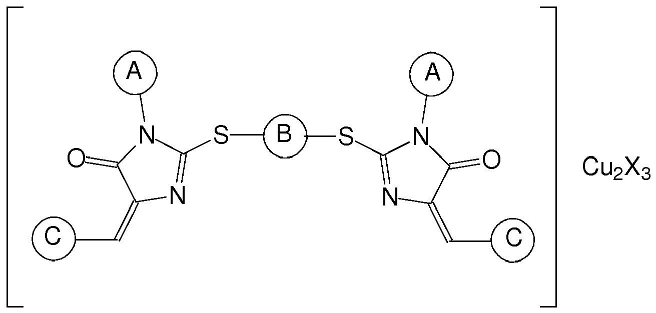 where substituent A selected from group including aryl and alkyl substitutes, condensed aromatic groups, cyclopentyl, cyclohexyl, aliphatic substituent's, aliphatic substitutes with double bonds, aliphatic substitutes with triple bonds, CH₃NH- group, C₂H₅O(O)C-group, five membered heterocycles with one nitrogen atom, five membered heterocycles with two nitrogen atoms, six membered heterocycles, substitute B is absent or aliphatic substitute, substitute C is heteroaromatic substitute , attached to imidazol-4-one derivative via carbon atom and selected from the group including, 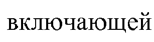 five membered saturated monocyclic heterocycles with 1, 2, 3 heteroatoms in cycle, selected from the group including N, O or S, 6-membered unsaturated monocyclic heteroaromatic substitutes with 1, 2, 3 heteroatoms in cycle, selected from the group including N, O or S, 8-, 9- or 10-membered unsaturated bicyclic heteroaromatic substitutes with 1, 2, 3 heteroatoms in cycle, selected from the group including N, O or S, X presents chloride Cl or nitrate NO₃.

2. Coordination compounds according to claim 1, wherein said that substituent A is unsubstituted or monosubstituted, or disubstituted with aryl group, in this case substitute R in aryl fragment choose from the group including halogens and alkyl groups.

3. Coordination compounds according to claim 1, wherein said that substituent A is unsubstituted or monosubstituted, or disubstituted with condensed aryl group, in this case substitute R in aryl fragment choose from the group including halogens and alkyl groups.

4. Coordination compounds according to claim 1, wherein said that substituent A was selected from the group including phenyl C₆H₅-, 3-chloro-4-fluorophenyl 3-Cl-4-F-C₆H₃-, 4-carbethoxyphenyl 4-C₂H₅O(O)CC₆H₄-, methyl CH₃-, allyl CH₂=CHCH₂-, 2-antranyl, propyl C₃H₇-

5. Coordination compounds according to claim 1, wherein said that substituent B was selected from the groups including 1,2-ethandiyl -(CH₂)₂-, 1,3-propanediyl -(CH₂)₃-, 1,4-buthanediyl -(CH₂)₄-, 1,6-hexanediyl -(CH₂)₆-, 1,10-decanediyl -(CH₂)₁₀-.

6. Coordination compounds according to claim 1, wherein said that substituent C was selected from the group including 2-quinolyl, 2-pyridyl, 1-methyl-2-imidazolyl, 4-methyl-5-imidazolyl, 5-imidazolyl, 2-imidazolyl, 1,5-dimethyl-3-pyrazolyl, 1,5-diphenyl-3-pyrazolyl.

7. Method for the preparation of coordination compounds on the basis of 2-thiohydantoines - telomerase inhibitors, according to claims 1-6, including following steps: solution of imidazol-4-one in DCM were slowly added to the solution of copper salt in methanol or acetonitrile, the precipitation of the coordination compound occurs.

8. Method according claim 6 wherein said that copper salt was selected from copper chloride and copper nitrate.


SEQUENCE LISTING

<110>  Gosudarstvennoe Uchebno-nauchnoe Uchrezhdenie Khimichesky
       Fakultet MGU Im. M.V. Lomonosova
       Mazhuga, Aleksandr Georgievich
       Zvereva, Marija Ehmil'evna

<120>  TELOMERASE INHIBITORS AND A METHOD FOR THE PREPARATION THEREOF

<130>  PCT/RU2011/000223

<140>  EP11766224.7
<141>  2012-11-08

<150>  RU2010113946
<151>  2010-04-09

<160>  2

<170>  PatentIn version 3.5

<210>  1
<211>  18
<212>  DNA
<213>  Artificial sequence

<220>
<223>  primer TS

<400>  1
aatccgtcga gcagagtt                                                    18


<210>  2
<211>  30
<212>  DNA
<213>  Artificial sequence

<220>
<223>  primer ACX

<400>  2
gcgcggctta cccttaccct taccctaacc                                       30